**Europäisches Patentamt**

**European Patent Office**

**Office européen des brevets**

(19)

(11) Veröffentlichungsnummer: **0 206 192**
**B1**

(12) **EUROPÄISCHE PATENTSCHRIFT**

(45) Veröffentlichungstag der Patentschrift:
**20.04.88**

(51) Int. Cl.⁴: **C 07 C 15/40**, C 07 C 5/333

(21) Anmeldenummer: **86108177.6**

(22) Anmeldetag: **14.06.86**

(54) **Verfahren zur katalytischen Dehydrierung von Alkylaromaten.**

(30) Priorität: **19.06.85 DE 3521765**

(43) Veröffentlichungstag der Anmeldung:
**30.12.86 Patentblatt 86/52**

(45) Bekanntmachung des Hinweises auf die Patenterteilung:
**20.04.88 Patentblatt 88/16**

(84) Benannte Vertragsstaaten:
**AT BE CH DE FR GB LI NL**

(56) Entgegenhaltungen:
**Keine Entgegenhaltungen.**

(73) Patentinhaber: **BASF Aktiengesellschaft,
Carl-Bosch-Strasse 38, D-6700 Ludwigshafen (DE)**

(72) Erfinder: **Ambach, Eberhard, Dr., Carl-Bosch-Strasse 7,
D-6703 Limburgerhof (DE)**
Erfinder: **Biffar, Werner, Dr., Fontanesistrasse 11,
D-6710 Frankenthal (DE)**
Erfinder: **Hoelderich, Wolfgang, Dr., Mannheimer
Strasse 18 c, D-6710 Frankenthal (DE)**
Erfinder: **Irgang, Matthias, Dr., Andreas-Hofer-Weg 41,
D-6900 Heidelberg (DE)**
Erfinder: **Mross, Wolf Dieter, Dr.,
Anselm-Feuerbach-Strasse 21, D-6710 Frankenthal (DE)**

**Beschreibung**

Die Erfindung betrifft ein Verfahren zur katalytischen Dehydrierung von Alkylaromaten.

Vinylaromaten, insbesondere Styrol, sind wichtige Ausgangsstoffe für die Herstellung von Kunststoffen, synthetischen Harzen und verschiedenen Kautschukarten.

Im allgemeinen werden sie technisch durch Überleiten von teilgesättigten Kohlenwasserstoffen, z.B. Ethylbenzol, zusammen mit Wasserdampf über einen Festbettkatalysator bei Temperaturen zwischen 500 und 700° C hergestellt. Bei dem Verfahren gibt es je nach Art der Wärmezuführung eine isotherme und eine adiabatische Ausführungsform.

Eine ausführliche Beschreibung der üblichen Verfahren findet sich im Kunststoff-Handbuch, Bd. V, Polystyrol, Karl-Hanser-Verlag, auf den Seiten 39 bis 47 (1969). Dort ist auch eine Vielzahl von geeigneten Dehydrierkatalysatoren beschrieben, die im wesentlichen aus Gemischen verschiedener Metalloxide bestehen. Die technisch wichtigsten Katalysatoren basieren auf Eisenoxid als Hauptkomponente, der oxidbildende Alkalisalze sowie strukturstabilisierende, aktivitäts- und selektivitätssteigernde Metallverbindungen zugemischt werden.

Die Katalysatoren werden üblicherweise in Form von gepressten, zylindrischen Strängen, selten als Tabletten oder Kugeln eingesetzt.

Die Teilchengrösse der Katalysatorpartikel variiert zwischen 1,5 und 19 mm. Beim isothermen Verfahren muss infolge des hohen Druckverlustes in den Röhren des Dehydrierreaktors die Teilchengrösse mindestens 5 mm betragen. In den Radialreaktoren, die beim adiabatischen Verfahren eingesetzt werden, können in den kürzeren Festbettschüttungen, im allgemeinen Katalysatorstränge mit 2,5 bis 5 mm Durchmesser verwendet werden.

Aus kinetischen Gründen ist eine möglichst kleine Katalysatorteilchengrösse wünschenswert, da bei der hochselektiven Dehydrierreaktion nur bei kurzen Verweilzeiten im Katalysatorpellet, d.h. geringen Diffusionswegen der Edukte und Produkte zur aktiven Katalysatoroberfläche und zurück in die Gasphase, maximale Ausbeuten an gewünschtem Dehydrierprodukt erhalten werden. In der DE-OS 25 44 185 wird diesem Anspruch durch Wahl besonderer Pelletgeometrien, z.B. Zylinderring und Sternstrang Rechnung getragen.

Wenngleich bei Verwendung derart geformter Katalysatorteilchen erhöhte katalytische Wirkungen erzielt wurden, bleibt aufgrund der hohen technischen Bedeutung des Styrol- und anderer ähnlicher Dehydrierverfahren die Aufgabe eine weiteren Verbesserung von Aktivität und Selektivität der Dehydrierkatalysatoren durch kinetisch günstigere Formgebung bestehen.

Es wurde nun gefunden, dass man bei einem Verfahren zur katalytischen Dehydrierung von Alkylaromaten zur Herstellung von Vinylaromaten bei erhöhter Temperatur in Gegenwart von Wasserdampf und Dehydrierungskatalysatoren besonders vorteilhafte Bedingungen und Ergebnisse erzielt, wenn man die Dehydrierkatalysatoren in Wabenform einsetzt.

Die Verwendung von Wabenkörpern in der Technik an sich ist vielfältig und reicht vom Einsatz als Filtermaterial, Trockenmittel für Gase, Füllmaterial in Waschtürmen, Destillationskolonnen und als Hitzeschilde bis zur heterogenen Katalyse. Im letzteren Falle beschränkte sich bisher der technische Einsatz von Wabenkörpern auf die Entgiftung von Verbrennungsabgasen und die katalytische Verbrennung (DE-AS 20 45 488). Zur Herstellung von Wabenkörpern werden in der Regel keramische Matrialien verwendet, wobei in erster Linie Cordierit und Mullit zu nennen sind. Aber auch der Einsatz von anderen typischen Katalysatorträgermaterialien wie Aluminiumoxid, Magnesiumoxid, Titandioxid, Zirkondioxid, Zirkonsilikat, Kieselsäure und Magnesiumaluminiumoxide ist bekannt (US-PS 3 112 184, DE-OS 22 54 563 und DE-OS 21 52 498). Diese Wabenkörper werden mit einer katalytisch aktiven Masse oder einem sogenannten «wash coat» beschichtet, auf den die eigentlich katalytisch aktive Komponente aufgetränkt wird.

Zur Herstellung der erfindungsgemässen Dehydrierungskatalysatoren kann man solche vorgeformte keramische Träger in Wabenform verwenden und diese mit einem aus aktiver Katalysatormasse bestehenden Schlicker beschichten.

Man kann aber vorteilhaft auch die Dehydrierungskatalysatoren als Vollkatalysatoren einsetzen, d.h. Wabenkörper werden vollständig aus aktiver Katalysatormasse hergestellt und eingesetzt.

Die erfindungsgemässe Dehydrierungskatalysatoren können verschiedene Wabenformen aufweisen. Die Grundfläche kann kreisförmig, oval oder polygonal sein. Die Länge kann ein Vielfaches des Querschnitts erreichen. Die Körper sind von einer Vielzahl parallel verlaufender Kanäle durchzogen, deren Grundfläche ebenfalls kreisförmig, oval, geschwungen oder eckig sein kann. Entscheidend für einen niedrigen Druckverlust ist der freie Querschnitt der Anströmfläche, der über 40% erreichen soll. Die Festigkeit des Wabenkörpers limitiert den freien Querschnitt.

Auch den Wabenkörpern ähnliche Formkörper können eingesetzt werden, deren Kanäle nicht parallel zur zentralen Achse sondern schräg dazu durch den Körper verlaufen im Prinzip des statischen Mischers, z.B. Kerapak®-Formen. Mit diesen Formkörpern wird die Quervermischung der Reaktionsgase verbunden mit einem erhöhten Wärmetausch zwischen Reaktorwand und Innenzone.

Die äusseren Dimensionen der Formkörper erreichen üblicherweise bis zu 150 mm Kantenlänge der Grundfläche bei Quadern und etwa gleichen Durchmesser der Grundfläche bei Zylindern. Die Länge der Körper über den Grundflächen kann über 1 000 mm liegen. Die lichte Weite der inneren Kanäle kann von 0,1 bis 20 mm, die Stärke der inneren Trennwände zwischen 0,1 und 5 mm variieren.

Um den vorgenannten kinetischen Anforderungen an den Dehydrierungskatalysator zu entsprechen, muss auf eine grosse geometrische Oberfläche pro Volumeneinheit geachtet werden. Dies wird durch Wabenkörper mit einer hohen Anzahl von Kanälen (z.B. $\geq 7/cm^2$) erreicht. Damit verbunden kann die Dicke der inneren Trennwände auf $\leq 2,0$ mm zurückgenommen werden. Die kurzen Diffusionswege der Reaktanden im Katalysatormaterial haben eine deutliche Steigerung der Selektivität zur Folge.

Durch die Länge der Wabenkörper, die Oberflächenrauhigkeit und die Gasgeschwindigkeit sind weitere Möglichkeiten für die Optimierung der Reaktion mit an sich bekannten Massnahmen gegeben. Der Synthesegasstrom wird im allgemeinen mit Durchsätzen von 0,1 bis 4 m/sec, insbesondere 0,5 bis 3 m/sec durch die Wabenkörper geführt.

Da der Synthesegasstrom praktisch keine Staubpartikel enthält, ist ein Zusetzen der Kanäle insbesondere in den Ecken — auch bei Winkeln $<90°$ — nicht zu befürchten. Mechanisch bedingter Katalysatorabrieb wird darüberhinaus weitgehend unterbunden, da die für Pellet-Festbettschüttungen typischen Bewegungen im Gasstrom nicht auftreten.

Beim Einsatz im isothermen Reaktor können die Wabenkörper der Rohrgeometrie angepasst in den Rohren übereinandergesetzt werden. Die Durchmesser der Wabenkörpergrundflächen sollten dabei infolge des notwendigen Wärmeaustausches mit dem Heizmedium auf der Rohraussenseite nicht zu gross gewählt werden ($\leq 100$ mm). Die vergleichbar geringe Wärmeleitfähigkeit der oxidischen Massen kann durch Verwendung von mit Aktivmasse beschichteten Metallwabenkörpern angehoben werden. Andererseits können zwischen die Wabenkörper kleine statische Mischer eingesetzt werden, die selbst katalytisch inert, aber auch aktiv sein können.

Auch ein unmittelbarer Wärmetausch zwischen Heizmedium und Synthesegas ist möglich. Hierzu werden beide Komponenten in getrennten, jedoch parallel laufenden Kanälen durch den Wabenkörper geführt. Wird Wasserdampf als Heizmedium eingesetzt, können ohne weiteres auch poröse Wabenkörpermaterialien zu diesem Zweck eingesetzt werden. Aktive Katalysatormasse eignet sich dann auch für die Herstellung solcher Wabenkörper.

Für den Einsatz in adiabatischen Reaktoren empfiehlt sich ein modularer Katalysatoreinbau. Dazu werden die Formkörper in Metallkästen zu sogenannten Modulen zusammengesetzt und in den Reaktor geschichtet. Nach Durchtritt durch eine Modullage im Reaktor kann das Reaktionsgas wieder aufgeheizt werden und über eine weitere Modullage geleitet werden. So können mehrere Modullagen mit Zwischenaufheizung hintereinander geschaltet werden, so dass auch bei adiabatischer Fahrweise ein leicht ansteigendes Temperaturprofil mit fortschreitendem Umsatz erzielt wird.

Problematisch insbesondere beim adiabatischen Dehydrierprozess ist bei den bekannten Verfahren der Alkaliverlust in der Eingangszone der Katalysatorschüttung. Mit zunehmender Reaktionszeit setzt sich dieser zur Festbettmitte weiter fort. Damit verbunden ist eine erhebliche Minderung der Katalysatoraktivität und -selektivität, was letzlich den Austausch des gesamten Katalysatormaterials notwendig macht.

Das erfindungsgemässe Verfahren bietet bei modularem Katalysatoreinbau den konstruktiven Vorteil, dass man lediglich die Modullagen austauschen kann, die tatsächlich in ihren katalytischen Eigenschaften stark nachgelassen haben.

Neben dem Einsparen von Katalysatormaterial wird bei diesem Verfahren auch ein grosser Zeitgewinn infolge einfachen und raschen Modulwechsels möglich.

Der geringe Druckverlust, den die Wabenkörper verursachen, erlaubt eine deutliche Steigerung der Katalysatorbelastung. Damit wird eine deutliche Absenkung des Katalysatorvolumens möglich, was eine kompaktere Reaktorbauweise zulässt. Etwas kürzere Standzeiten, die dabei gegebenenfalls auftreten könnten, sollten infolge der bereits erwähnten Möglichkeit des raschen Modulaustauschs nicht wesentlich stören.

Zusammenfassend bietet der Einsatz von Katalysatoren in Form von Wabenkörpern folgende technische Vorteile:

a) Die günstigen kinetischen Voraussetzungen — grosse Oberfläche bei kurzen Diffusionswegen im Porensystem —, die zu einer deutlichen Aktivitäts- und Selektivitätssteigerung führen;

b) der geringe Druckverlust über das Katalysatorbett, der höhere Mengenströme pro Katalysatorvolumen und auch Reaktionen unterhalb Atmosphärendruck zulässt;

c) ein modularer Einsatz von Wabenkörpern erlaubt den raschen Ein- und Ausbau des Katalysators, auch schneller Katalysatorteilwechsel ist möglich;

d) die in einer Festbettschüttung auftretenden Pelletbewegungen unterbleiben, wodurch ein mechanischer Abrieb weitgehend unterbunden wird;

e) durch die handliche Form das Katalysators wird Transport, Aufarbeitung oder Regenerierung ausserhalb des Reaktors und die endgültige Entsorgung und Endlagerung des Katalysators erleichtert, insbesondere wenn bei einer anderen Aufarbeitung gesundheitsgefährdende Stäube auftreten können.

Die Vorteile der Verwendung von Wabenkörpern zur Dehydrierung organischer Verbindungen werden besonders deutlich bei einem Vergleich von Wabenkörpern und Katalysatorsplitt mit einer für technische Einsatzzwecke nicht verwertbaren Körnung, die der Stärke der inneren Zellwände des Wabenkörpers entspricht.

Bei einem Vergleichstest wird die überraschend höhere Aktivität und Selektivität der Wabenkörper deutlich.

Die nachfolgenden Beispiele sollen das erfindungsgemässe Verfahren verdeutlichen.

*Beispiel 1:*

Herstellung eines Vollkatalysators in Wabenform

10 000 g α-FeOOH, 1596 g $K_2CO_3$, 190 g $Cr_2O_3$, 146 g $V_2O_5$, 115 g $WO_3$ und 300 g Stärke werden gemischt und mit Wasser angemaischt. Nach 3 h Knetzeit wird die plastische Masse zu Wabenkörpern extrudiert. Die quadratische Grundfläche der erhaltenen quaderförmigen Körper hatte Kanten von 5 cm Länge. Die Länge der Quader beträgt 40 cm. Die 121 inneren Kanäle haben eine quadratische Stirnfläche mit 0,36 cm Kantenlänge. Die Dicke der inneren Trennwände beträgt 0,55 mm. Die Wabenkörper werden 2 Tage bei Raumtemperatur und einen weiteren Tag bei ansteigender Temperatur bis 150° C getrocknet. Anschliessend werden die Formkörper 5 h bei 500° C und 1 h bei 850° C calciniert.

*Beispiel 2:*

Herstellung eines Katalysators durch Beschichten eines wabenförmigen Trägers aus keramischem Material mit einer Aktivmasse

Ein strukturierter und gewickelter Wabenkörper aus keramischem Material mit einem Durchmesser der Grundfläche von 47 mm wird mit einem Schlicker, hergestellt durch Aufschlämmen der Aktivkomponenten mit der Zusammensetzung nach Beispiel 1 in Wasser, beschichtet. Nach eintägigem Trocknen bei Raumtemperatur wird die Masse 16 h bei 150° C getrocknet. Das Calcinieren erfolgt in zwei Stufen: 5 h 500° C und 1 h 850° C.

*Vergleichsbeispiel 3:*

Zum Vergleich werden aus einer Knetmasse gemäss Beispiel 1 Vollstränge mit 6 mm Durchmesser und ca. 10 mm Länge geformt. Die Stränge werden 3 h bei 150° C und 1 h bei 900° C getrocknet und calciniert.

*Vergleichsbeispiel 4:*

Für einen Vergleichstest wird ein Teil der Stränge aus Beispiel 3 zerkleinert und eine Splittfraktion von 0,75 bis 1,25 mm Korngrösse ausgesiebt.

Die Tests der Katalysatoren erfolgen in elektrisch beheizten Modellreaktoren. Die Rohrgeometrie des Reaktors wird dem Formkörper angepasst. Der Test der Stränge aus Beispiel 3 erfolgt in dem Modellrohr, das auch für den Wabenkörper von Beispiel 2 verwendet wurde. Das Katalysatorvolumen beträgt jeweils 1 Liter. Die Belastung wird auf 1 Liter fl. Ethylbenzol pro Liter Katalysator und Stunde eingestellt. Das Dampf/Ethylbenzol-Gewichtsverhältnis beträgt stets 1,0.

Der Test des Splitts erfolgt unter gleichen Zulaufbedingungen in einem kleinen Laborreaktor mit 6 mm Innendurchmesser mit Salzbadheizung.

In der nachfolgenden Tabelle sind die Testergebnisse zusammengefasst. Die gezeigten Analysenwerte werden nach 100 h Laufzeit bestimmt.

| Katalysator nach Beispiel bzw. Vergleichsbeispiel | Temperatur [°C] | Ethylbenzol-Umsatz [%][1] | Styrol-Selektivität [%][2] |
|---|---|---|---|
| 1 | 610 | 62,9 | 94,8 |
| 2 | 607 | 64,2 | 94,9 |
| 3 | 623 | 59,5 | 93,7 |
| 4 | 615 | 60,5 | 93,9 |

[1] Umsatz:
$$\frac{Ethylbenzol_{vor\ Katal.} - Ethylbenzol_{nach\ Katal.}}{Ethylbenzol_{vor\ Katal.}} \cdot 100\ [\%]$$

[2] Selektivität:
$$\frac{Styrol\ im\ organischen\ Austrag}{Umgesetztes\ Ethylbenzol} \cdot 100\ [\%]$$

**Patentansprüche**

1. Verfahren zur katalytischen Dehydrierung von Alkylaromaten zur Herstellung von Vinylaromaten bei erhöhter Temperatur in Gegenwart von Wasserdampf und Dehydrierungskatalysatoren, dadurch gekennzeichnet, dass man die Dehydrierungskatalysatoren in Wabenformen einsetzt.

2. Verfahren zur katalytischen Dehydrierung nach Anspruch 1, dadurch gekennzeichnet, dass der Katalysator aus Aktivmasse besteht.

3. Verfahren zur katalytischen Dehydrierung nach Anspruch 1, dadurch gekennzeichnet, dass der Katalysator aus einem keramischen Träger in Wabenform besteht, auf den die Aktivmasse aufgebracht ist.

4. Verfahren zur katalytischen Dehydrierung nach Anspruch 1 bis 3, dadurch gekennzeichnet, dass Ethylbenzol zu Styrol dehydriert wird.

**Claims**

1. A process for the catalytic dehydrogenation of an alkylaromatic for the preparation of a vinylaromatic at elevated temperature in the presence of steam and a dehydrogenation catalyst, wherein the dehydrogenation catalyst is used in the form of a honeycomb.

2. A process for catalytic dehydrogenation as claimed in claim 1, wherein the catalyst consists of active material.

3. A process for catalytic dehydrogenation as claimed in claim 1, wherein the catalyst consists of a ceramic carrier in the form of a honeycomb, to which the active material is applied.

4. A process for catalytic dehydrogenation as claimed in claims 1 to 3, wherein ethylbenzene is dehydrogenated to styrene.

**Revendications**

1. Procédé pour la déshydrogénation catalysée de composés alkylaromatiques à température accrue, en présence de vapeur d'eau et de catalyseurs de déshydrogénation, pour la préparation de composés vinylaromatiques, caractérisé en ce que l'on utilise des catalyseurs de déshydrogénation en nid d'abeilles.

2. Procédé de déshydrogénation catalysée suivant la revendication 1, caractérisé en ce que le catalyseur est formé de matière active.

3. Procédé de déshydrogénation catalysée suivant la revendication 1, caractérisé en ce que le catalyseur est constitué d'un support céramique en nid d'abeilles, sur lequel est déposée la masse active.

4. Procédé de déshydrogénation catalysée suivant les revendications 1 à 3, caractérisé en ce qu'il est mis en œuvre pour la déshydrogénation de l'éthylbenzène en styrène.